# EUROPEAN PATENT SPECIFICATION

(11) **EP 1 886 631 B1**
(45) Date of publication and mention of the grant of the patent: **23.11.2016**
(21) Application number: 06743464.7
(22) Date of filing: 05.05.2006
(51) Int. Cl.: A61B 17/06, A61F 2/00

(54) **SUBURETHRAL SLING FOR THE SURGICAL TREATMENT OF FEMALE URINARY INCONTINENCE AND SURGICAL INSTRUMENTS USED TO POSITION SAME**
SUBURETHRALE SCHLINGE FÜR DIE CHIRURGISCHE BEHANDLUNG VON HARNINKONTINENZ BEI FRAUEN UND OPERATIONSINSTRUMENTE ZU IHRER POSITIONIERUNG
RUBAN SOUS-URETRAL POUR LE TRAITEMENT CHIRURGICAL DE L'INCONTINENCE URINAIRE FEMININE ET INSTRUMENTS CHIRURGICAUX DESTINES A LA MISE EN PLACE DE CE RUBAN

(30) Priority: 06.05.2005 ES 200501090 P
(43) Date of publication of application: 13.02.2008
(73) Proprietor: Gambini Ricapa, Juan, 46008 Valencia (ES)
(72) Inventor: Gambini Ricapa, Juan, 46008 Valencia (ES)
(74) Representative: Sahuquillo Huerta, Jesús
(86) International application number: PCT/ES2006/000214
(87) International publication number: WO 2006/136625

(56) References cited:
- ES-A1- 2 226 564
- US-A1- 2001 053 916
- US-A1- 2003 004 523
- US-A1- 2004 144 395
- US-A1- 2004 243 166

## Description

As stated in the title of this descriptive specification, the present invention relates to improvements introduced into invention patent No. P-200301134 (published as ES 2226564) for:
suburethral sling for the surgical treatment of female urinary incontinence, thanks to which stress urinary incontinence in women manages to be corrected with great ease and effectiveness, and for both the surgical device with which such improvements are achieved as well as the advantageous operating method that can be carried out.

The suburethral sling or mesh for the surgical correction of female stress urinary incontinence, whose characteristics and properties are described in the main invention patent P-200301134, is a notable advance on the slings or meshes that are currently used for this purpose, due both to the fact that its length is shorter since it is only located beneath the urethra and is anchored to the endopelvic or urethropelvian fascia and also on account of its characteristics presented by the edges of its contour: those of the ends are going to allow this mesh to penetrate and not come away from the place where it has been positioned, acting by way of an arrow or harpoon, so that it does not become displaced and attached to adjacent tissues; and those of the central zone are smooth in order to avoid irritation of the mid-urethra and vesical neck when clasped by the band or mesh materialising its structure, and the black silk thread which traverses it, which, as well as marking the midpoint, also serves to extend the mesh in the event that it becomes rolled up so that it remains correctly positioned.

US 2004/0144395 discloses an instrument in the form of a connector for implantation of a suburethral mesh.

It is also an object of the invention to provide the instruments for the implantation of this suburethral mesh in accordance with claims 2 and 3 which has to be carried out in order to correct stress urinary incontinence, being an operation that is very simple, easy to perform and minimally traumatic.

The application of this suburethral sling to a patient implies surgical treatment of stress urinary incontinence and has the aim of correcting alterations of the pelvic girdle, achieving the stabilisation, securing and raising of the urethra and vesical neck in order to prevent leaks of urine during stresses. The particular features offered by the present invention are: a) the actual characteristics of the suburethral sling or mesh described in the main invention patent and the structural improvements that are now proposed; b) the instruments for its implantation; and c) the simplicity of the technique which also makes its positioning instruments simple, reusable, low economic cost, and minimally invasive.

### PRIOR ART OF THE INVENTION

Stress urinary incontinence is understood as the involuntary loss of urine through the urethra, caused by an increase in intra-abdominal, and therefore intra-vesical, pressure, overcoming the urethral closing pressure, without contraction of the detrusor.

Woolin (J. Urol., 101: 545-546, 1969) interrogated 4000 childless women and found that 50.7% of them showed a certain degree of urinary incontinence to stresses. Stress urinary incontinence is produced by an alteration to the muscle-tendon structures of the pelvic girdle due, on the one hand, to the erect position of the female pelvis which has to support the forces produced in its interior and as a consequence of the stresses, they facilitate the descent of the urethral-vesical union, with tissue aging.

There exist two types of stress urinary incontinence that are most frequent in women: that due to urethral hypermobility and that due to intrinsic sphincter deficiency of the urethra.

Stress urinary incontinence due to urethral hypermobility is described in clinical terms as the demonstration of displacement of the anterior vaginal wall and descent of the urethra during the stress, with the urethral closing pressure being normal. Here, the mechanisms of urethral closure at the level of the mid-urethra are altered due to the loss of support provided by the pubo-urethral ligaments, the suburethral anterior vaginal wall and the loss of the function and insertion of the muscles of the pelvic girdle (pubo-coccygeal muscles).

Stress urinary incontinence due to intrinsic sphincter deficiency is produced by the decrease in the occlusive force or urethral closure as a consequence of insufficient urethral coaptation and compression, very often associated with the permanent opening of the vesical neck and its displacement during stresses. The stabilisation of the cervico-urethral union, which is responsible for providing backing and support for urethral compression, and which also actively compresses the proximal urethra during stresses, is altered and as a consequence of the failure of these mechanisms leakage of urine takes place under minimal stresses such as the simple act of walking or standing on two feet.

According to these concepts, treatment has to be aimed at correcting these alterations. Nevertheless, procedures are currently used which are limited to just correcting urethral hypermobility, these techniques being very invasive and strict learning is required for carrying them out which is done only by experts. Such is the case with correction by laparoscopy through the abdominal wall which, though the entry points are very small, requires very costly instruments and the operating time usually lasts between 80 and 120 minutes, it is always done with a general anaesthetic, and cases of vesical and vascular lesion have occurred making it necessary to perform a laparotomy in order to solve these complications. Other techniques are performed through the vagina and abdomen, passing a mesh from the vagina until withdrawing it via the skin of the abdominal wall free of tension, for which a general, local-regional or local anaesthetic is used; in these cases a large dimension mesh is used of 50 or 60 cm in length whose ends are attached to some needles with a special device for directing those needles blind and thus avoid lesion to the urethra and to the bladder; they penetrate from the vaginal zone (suburethral), traverse the urogenital diaphragm, endopelvic fascia, cross towards the midpoint, pass through the fascia of anterior straight muscles of the abdomen until exiting through the skin behind the pubic bone in its central zone. Here, complications have been described such as urethral, vesical and intestinal perforation and perforation of the external iliac artery, which are sometimes very serious and it is always essential to perform a cystoscopy in order to discard vesical perforation, so that the time spent in surgery is extended even further. The surgical material for doing this is costly and requires meticulous learning, both for the surgical technique and for performing the cystoscopy.

As a consequence of the presence of these lesions, other techniques have appeared which, instead of securing the urethra upwards (suprapubic region), do so downwards towards the sides by way of a board, in other words to prevent the lesions described above, and they pass the needle with the mesh towards the lateral face of the vagina, searching for the obturator hole, which they perforate until exiting through the skin of the internal face of the thigh. Passing through the hole of the obturator are the nerve and the obturator artery, and, as the needles are directed blind, lesions have occurred to these structures and there have also been delayed complications such as inflammation, fibrosis and retractions caused by the mesh on its route from the vaginal zone to the internal face of the thighs, causing serious disturbance to the movement or opening of them.

For the surgical treatment of incontinence due to urethral intrinsic sphincter deficit, techniques have been described for the suspension of the vesical neck via the combined vaginal and abdominal route, using long meshes or bands of an un-reabsorbable material, or organic or synthetic reabsorbable materials, which pass from the vagina (suburethral space) to the abdomen, with a prior transverse incision of from 6 to 10 cm of the abdominal skin from behind the pubis, the dissection is deepened until it reaches the fascia of the anterior straight muscles, where the ends of the mesh are sutured, the band sometimes being left very tight which causes acute or chronic urinary retention, or a device made of a synthetic and metallic material is also positioned in order to regulate the tension of the mesh. In this regard, complications have been described, due both to the mesh sutured there and to the device positioned there, whose drawbacks are: as far as the mesh is concerned, intolerance, inflammation, infection and fistulisation; in terms of the adjustable device, there is a frequent appearance of intolerance causing large seromas that take a long time to clear, infections and disturbances in the zone of the implant, in addition to the fact that the material used for doing this is very costly. Vesical perforation when passing the needles is relatively frequent in these procedures, making it necessary to perform a cystoscopy, which means that the time spent in surgery is extended due to the systematic use of the cystoscope.

### DESCRIPTION OF THE INVENTION

In general terms, the suburethral sling described in the main invention patent P200301134 (published as ES2226564) and the improvements introduced pursuant to the present invention, on account of both the actual structure and configuration of the mesh and the novelty of its implantation instruments, make it possible to use a new method conceived for the surgical treatment of female stress urinary incontinence, which is given the term: Endopelvic Free Anchorage.

In the case of correcting stress urinary incontinence due to urethral hypermobility, the suburethral sling is positioned beneath the mid-urethra, a small sagittal incision is made of about 2 cm in the anterior wall of the vagina. Mayo scissors are used to dissect and tunnel both paraurethral spaces until reaching the lower edge of the pubic bone. The implantation instrument for the suburethral sling that is used consists of a long curved clamp, of small calibre, with a conical tip, slightly round and containing an eye. The tip of the clamp is used to clasp the end of the mesh or band forming the suburethral sling, it is introduced though the dissected paraurethral space and behind the pubis and stuck to the latter by means of a gentle upwards pressure, the urogenital diaphragm is traversed until perforating the endopelvic fascia or urethropelvian fascia; the clamp securing the end of the mesh is opened and the latter remains trapped in the endopelvic or urethropelvian aponeurotic eye, the clamp is withdrawn, the same operation is repeated on the opposite side and the mesh is positioned free of tension, adhered to the suburethral tissue, fixing and stabilising the urethra.

In cases of stress urinary incontinence due to intrinsic sphincter deficit, the suburethral mesh or band is positioned in the proximal urethra. To achieve this, an implantation instrument is used consisting of a long curved needle, of small calibre, with an eye at its end. Approximately 1 cm from the end of the suburethral mesh, a monofilament thread is interlaced, whose length is going to allow it to pass from the vagina as far as outside the retropubic abdominal skin, the two ends of the thread pass through the eye of the needle via its convex part, until the end of the mesh is secured to the tip of the needle, the threads are fastened to a fastening device which is close to the control device or handle, so that the mesh does not become displaced when introduced along with the needle through the previously dissected paraurethral spaces, below and behind the pubis and stuck to the latter. By means of gentle pressure on the needle, the urogenital diaphragm is passed until perforating the endopelvic or urethropelvian fascia, and the end of the mesh remains anchored and trapped in the endopelvic or urethropelvian aponeurotic eye, and at that moment the two threads keeping the end of the mesh secured to the tip of the needle are released by opening the fastening device. This causes the threads to slide along the eye while the needle continues to be introduced around the back of the branch of the pubic bone in the vertical direction until its tip passes through the abdominal skin, only until the eye appears with the two threads, and via its concave face these threads are pulled until the loose ends pass from the vaginal zone, until they are withdrawn out of the abdominal skin from behind the publis; after that the eye is withdrawn and the positioning instrument is removed, with the same manoeuvre being performed on the opposite side and the vaginal incision is then sutured.

The threads appearing through the skin on each side of the suprapubic region will, when they are pulled upwards, serve to adjust the degree of tension required by the mesh until the urinary incontinence is corrected, whether this be by measuring the degree of cervico-urethral angle or by getting the patient to cough until she is continent, which is done during the operation or the following day when standing up, by means of a cough stress or Valsalva. One of the ends is then cut flush with the skin in order to not contaminate the inside of their route, the other one is pulled and the entire thread is extracted.

The suburethral sling of main invention patent P200301134 is materialised by a polypropylene monofilament band or mesh, whose central zone has both edges smooth to prevent irritation of the urethra and vesical neck when gripped by the band, but the rest of which is designed in the form of an arrow which, when introduced, cannot exit and which will serve to adhere strongly to the endopelvic fascia and adjacent tissues, remaining in the place where it is positioned.

The advantages of this technique using the suburethral sling with its positioning device which comprises one of the improvements contributed in accordance with the invention, compared to current devices for correcting stress urinary incontinence, are the following:
- There is no need to go deeply towards the most internal organs, so there is no danger of serious lesions.
- There does not exist any vesical perforation because the structure that is traversed is just the urogenital diaphragm and the endopelvic fascia, so there is no need to perform a cystoscopy.
- The route which the needle takes as far as reaching the skin is straight and therefore free of complications, while the other techniques cross it in order to exit at the central part of the retropubis.
- There is no risk of the appearance of inflammations and infections (cellulitis) or retractions in the subcutaneous cellular tissue since no foreign component has been implanted in this zone.
- The advantage of adjusting the tension of the mesh at the level of the urethropelvian fascia is the absence of urethral obstruction; when it is done at the level of the fascia of the straight muscles of the abdomen when the patient stands up, it distends and strangles the urethra, causing acute urinary retention requiring the mesh to be withdrawn; urinary retentions have also been produced when, with the passage of time, the patient increases her abdominal perimeter, whether due to a weight increase or due to abdominal muscular-aponeurotic relaxation, and the adjusting prosthesis that has been implanted will pull on the threads of the mesh compressing the urethra.
- Due to not having any metallic prosthesis in the abdomen, these patients can be subjected to explorations such as nuclear magnetic resonance, and they can be subjected to abdominal surgical operations at any moment, without any need to turn to anti-incontinence surgery.
- It is a technique that is simple, short, easy to carry out and minimally invasive.
- Minimum surgical material: a band of short dimensions, a curved positioning clamp with eye and a thin needle with eye, reusable and therefore much more economical.
- It can be carried out with a local anaesthetic.
- The operating time is 10 to 20 minutes, which implies greater economy in the use of the operating theatre, and it can therefore be regarded as short-stay major surgery.
- The occupancy time for a hospital bed is reduced, also leading to greater economy.
- Given its absence of morbidity, it can be applied to patients at high surgical risk.
- Excellent results, similar to current techniques of suburethral suspension.

In order to facilitate an understanding of the characteristics of the invention and forming an integral part of this specification, some sheets of plans are attached in which figures, on an illustrative rather than limiting basis, the following have been represented:

### BRIEF DESCRIPTION OF THE FIGURES

**Figure 1****.-** Is a schematic view in plan of a suburethral sling for the surgical treatment of female incontinence, described in main invention patent P200301134.
**Figures 1a to 1j****.-** Are respective views of different forms of embodiment of the sling, or parts thereof, in accordance with the invention.
**Figure 2****.-** Is a schematic view in plan of the instrument for implantation of the suburethral sling for cases of incontinence due to urethral hypermobility, with curved clamp and eye in the tip.
**Figures 2a and 2b****.-** Are respective partial views of the instrument of figure 2, in order to observe the slightly rounded tip and the location of the eye 1 cm from the end.
**Figure 3****.-** Is a schematic view in plan of an instrument for implantation in cases of incontinence due to intrinsic sphincter deficit and relapsing incontinence, having a curved needle with an eye in the tip.
**Figures 3a to 3j****.-** Are respective views of the surgical instrument of figure 3, or parts or zones thereof, with different embodiments of the device for securing the threads, and of the control device or handle.

### DESCRIPTION OF THE PREFERRED FORM OF EMBODIMENT

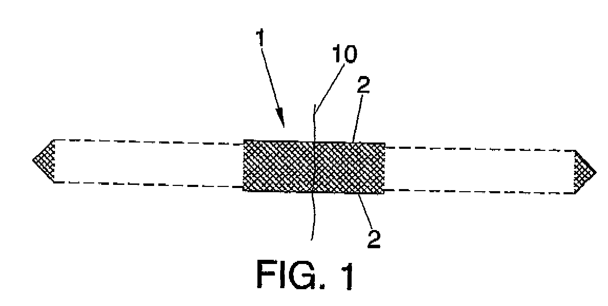

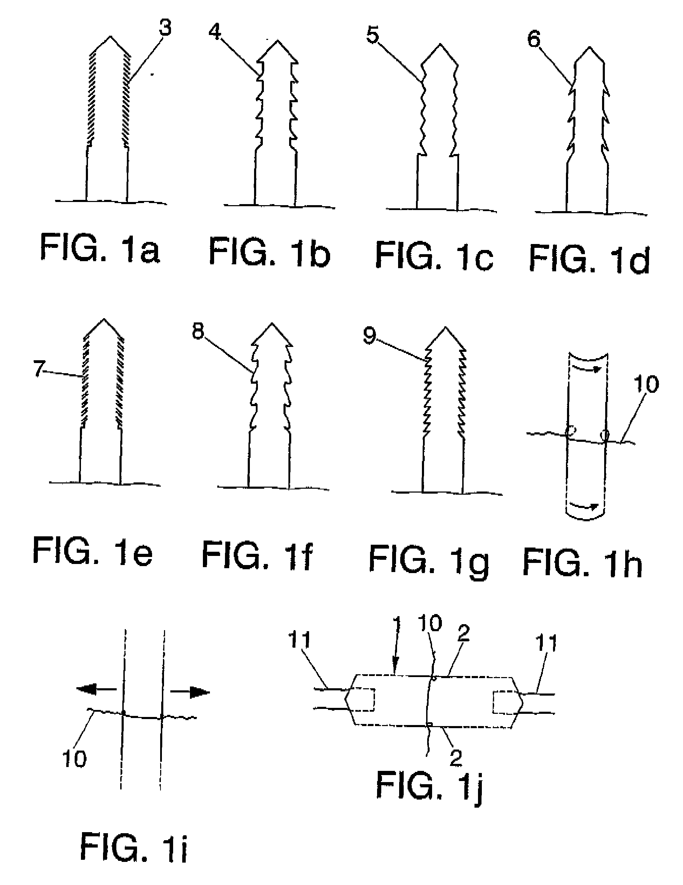

Making reference to the numbering adopted in figure 1, we can see that it concerns a mesh or band 1 described in the main invention patent and which can be made of a non-reabsorbable material such as polypropylene, nylon, polyethylene, polyester, fluoropolymers or other synthetic materials or synthetic reabsorbable materials such as polyglycolic acid, polylactic acid and other reabsorbable materials, monofilament or multifilament.

The edges of the central zone are smooth to prevent irritation of the tissues surrounding it, being referenced with the number 2. The sides of the ends of the band 1 are regular and are conceived thus with the aim that it does not move and will remain adhered to the adjacent tissues where it has been positioned, that it does not become displaced and does not exit from the site where it has been anchored, since the sides of the band will act as an arrow or harpoon, which penetrates but does not exit. As is seen in figures 1a to 1j, it can be fringed with the tips in an oblique direction and directed towards the centre of the band, like an arrow (reference 3 in figure 1 a); in the form of steps forming a toothed arrangement with separate teeth 4 (figure 1 b); toothed with continuous isosceles teeth 5 (figure 1c); toothed in an oblique direction towards the centre of the length of the band (referenced with 6 in figure 1d); cut in an oblique direction as observed in figure 1e and referenced with 7; undulating in the oblique direction as referenced with 8 in figure 1f; and in the form of continuous teeth in a sawtooth arrangement as shown in figure 1g, referenced with 9. As can be seen in figure 1h, in the middle of the sling or mesh there is a black silk thread interlaced which, as well as marking the midpoint of the length (as was considered in the main invention patent and is observed in figure 1), also serves to prevent it from folding on itself with its longitudinal edges approaching each other, as shown with arrows in this figure 1h and remain spread thus when the two ends are pulled, as observed in figure 1i.

For the proper positioning of the band or sling, and as can be seen in figure 1j, interlaced at from 7 to 15 mm from the two ends of the mesh 1 is a monofilament thread 11 which will serve as means of traction in order to adjust the tension of the mesh for correcting the urinary incontinence.

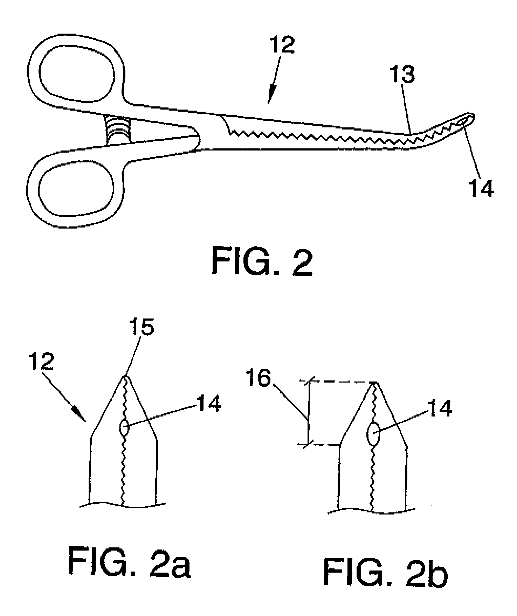

Making special reference now to figure 2, we can see how the implantation instrument of the suburethral mesh, for the treatment of stress urinary incontinence due to urethral hypermobility, which the invention proposes, is referenced in general with the number 12 and comprises a reusable metallic clamp, or disposable rigid plastic clamp, of about 15 to 20 cm in length, curved with an angle of approximately 30° to 45° in its distal quarter (zone indicated with 13), with an eye of 3 to 4 mm in diameter. It has a conical tip, slightly rounded, as shown in figure 2a referenced with 15, and with an eye of 3 to 5 mm in diameter and 1 cm from the end as designated with 16 in figure 2b.

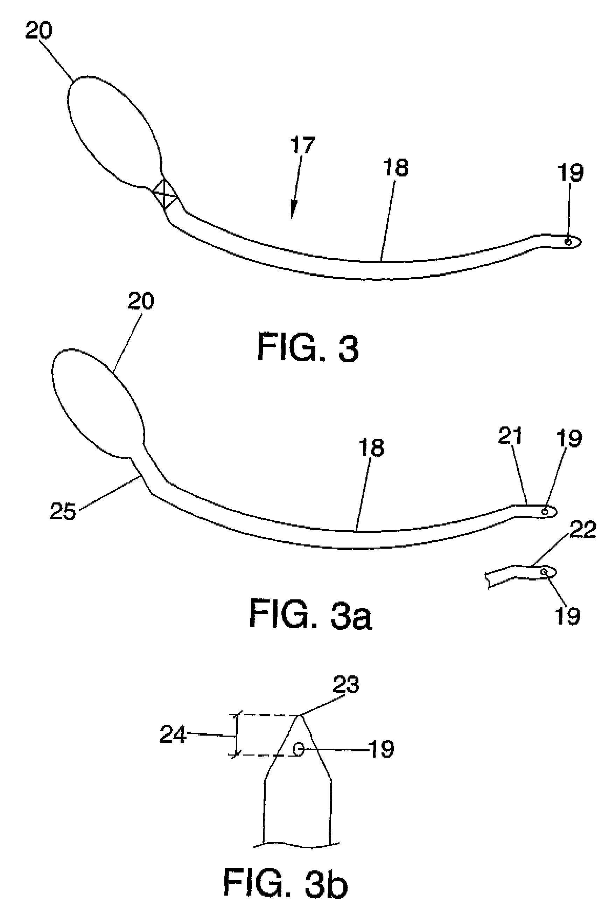

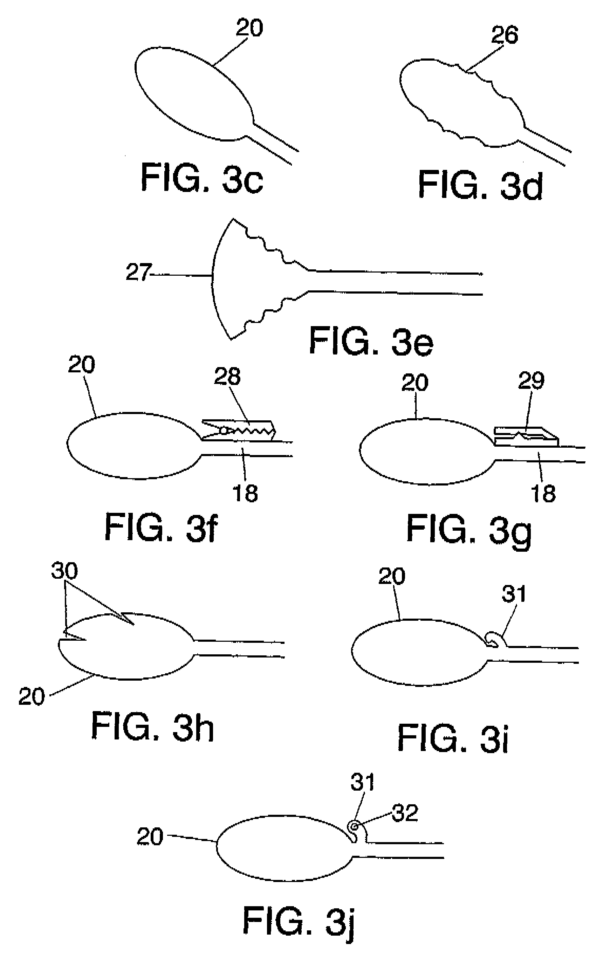

Shown in figure 3 is the instrument for implantation of the suburethral mesh for the surgical treatment of female urinary incontinence due to intrinsic sphincter deficit, relapsing incontinence or due to urethral hypermobility, being referenced in general with the number 17 and comprising a needle 18 of from 3 to 6 mm in diameter, metallic and reusable, or made of a disposable plastic material, with an eye 19 at its tip and with a sleeve or handle 20 at the opposite end. Next to the handle 20, a fixing system for the thread is mounted. This instrument 17 has a total length of 30 to 50 cm, the needle 18 being curved with an angle of from 20° to 60° (see figure 3a) and with its distal 3 to 4 cm having a straight trajectory, referenced with 21, or with a gentle curvature 22 at that end. Its tip is conical, slightly rounded (reference 23 in figure 3b) and with an eye 19 at 3 of 5 mm in diameter and from 5 to 10 mm from the tip, as indicated with 24 in figure 3b.

The control device or handle 20 of figure 3a presents its widening starting from a bent section 25 of about 40° to 60° with respect to the axis of the needle 18. It can be: cylindrical (not represented in the figures), from 8 to 12 cm in length; oval shape with a smooth contour as shown in figure 3c; provided with notches 26 (see figure 3d) for gripping with the fingers, or triangular form as seen in figure 3e referenced with 27 and also provided with notches.

At the level where the needle 18 ends and the sleeve or handle 20 starts (see figure 3f) a device 28 is found which serves to trap or fix the threads securing the ends of the suburethral sling, which serves to adjust the traction of the mesh and which in this case presents the form of a crocodile clip; though it can also have the form of a peg 29 (figure 3g); being defined by a slot 30 in the proximal part or in the distal part of the sleeve or handle (see figure 3h); have the form of a pivot 31 as shown in figure 3i, or include a hole 32 in this pivot 31 where the threads are secured, as shown in figure 3j.

## Claims

1. Suburethral sling for the surgical treatment of female urinary incontinence, arranged to secure the mid-urethra, vesical neck and proximal urethra, in order to prevent urine leaks during stresses and the sling comprising a monofilament or multifilament band or mesh (1) of polypropylene or other material, macroporous or similar, whose length corresponds to the endopelvic fascia to which it is anchored, its central zone presents both edges (2) smooth and a black silk filament (10) which crosses the band or mesh (1) centrally in order to mark its midpoint; and wherein, the rest of the band or mesh (1) being designed like an arrow tip; and wherein the band or mesh (1) is fringed with the tip in an oblique direction towards the center, in the form of steps or teeth either separate (4, 6) or contiguous (8, 9), of different angles, according to the case;
**characterized in that**
the band or mesh (1) includes a monofilament thread (11) interlaced at from 7 to 15 mm from both ends of the band or mesh (1), for being pulled and adjusting the tension of the mesh (1), correcting the stress urinary incontinence;
and wherein the black silk filament (10) remains anchored to the edges (2) in order to permit the transverse extension of the band or mesh (1);
and wherein the black silk filament (10) is arranged to prevent it from folding on itself with its longitudinal edges approaching each other and remain spread thus when the two ends are pulled.

2. Surgical instrument arranged to implant the suburethral sling of claim 1 in the treatment of stress urinary incontinence due to urethral hypermobility **characterized in that** it comprises a clamp (12) of about 15 to 20 cm in length for positioning of the band or mesh (1), the clamp (12) being curved with an angle comprised between 30º to 45º at its distal end (13), with a conical tip (15) and an eye (14) with a diameter comprised between 3 to 5 mm and 10 mm from its end (16).

3. Surgical instrument arranged to implant the suburethral sling of claim 1 in the treatment of female urinary incontinence due to intrinsic sphincter deficit or due to urethral hypermobility **characterized in that** it includes a reusable metallic or disposable plastic needle (17), of small caliber and curved, with its distal 3-4 mm bent in a form that is either straight (21) or slightly arched (22), with a conical tip (23) and with an eye (19), having its proximal end with a bulge or handle (20, 26, 27) for holding the needle.

4. The surgical instrument according to claim 3, wherein the arched needle has in proximity to the handle a bend of 40º to 60º with respect to its axis; and wherein it includes means (20, 26, 27) for trapping or fixing the thread (11) which secure the ends of the band or mesh (1).

5. The surgical instrument according to claim 4, wherein the means (20, 26, 27) for trapping or fixing the thread (11) have the form of a clip (28) or peg (29).

6. The surgical instrument according to claim 4, wherein the means for trapping or fixing (20, 26, 27) the thread (11) are defined by a slot (30) in a proximal or distal part of the control device handle (20), or an arched pivot (31) which can be provided with a hole (32).

## Patentansprüche

1. Suburethrale Schlinge für die chirurgische Behandlung weiblicher Harninkontinenz, die so angeordnet ist, dass sie die mittlere Harnröhre, den Blasenhals und die proximale Harnröhre sichert, damit es während einer Belastung nicht zu Harnverlust kommt, und wobei die Schlinge ein Monofil- oder Multifilband oder - netz (1) aus Polypropylen oder einem anderem Material, makroporös oder dergleichen, umfasst, dessen Länge der Beckeneingeweidefaszie entspricht, an der sie verankert wird, wobei beide Ränder (2) ihres mittigen Bereichs glatt sind, sowie einen Faden aus schwarzer Seide (10), der mittig über das Band oder Netz (1) verläuft und so seinen Mittelpunkt kennzeichnet; und wobei der Rest des Bands oder Netzes (1) wie eine Pfeilspitze ausgestaltet ist; und wobei das Band oder Netz (1) mit einem Saum versehen ist, mit der Spitze in einer schrägen Richtung auf die Mitte zu, in Form von Abstufungen oder Zähnen unter unterschiedlichen Winkeln, gegebenenfalls entweder separat (4, 6) oder zusammenhängend (8, 9); **dadurch gekennzeichnet, dass**
das Band oder Netz (1) einen monofilen Faden (11) aufweist, der bei von 7 bis 15 mm von beiden Enden des Bands oder Netzes (1) aus eingewebt ist, damit an ihm gezogen werden und er die Spannung des Netzes (1) anpassen kann und so die Belastungsharninkontinenz behoben wird;
und wobei der Faden aus schwarzer Seide (10) an den Rändern (2) verankert bleibt, damit die Querdehnung des Bands oder Netzes (1) ermöglicht wird;
und wobei der Faden aus schwarzer Seide (10) so angeordnet ist, dass er verhindert, dass es zusammenklappt und sich dabei seine Längsränder einander annähern, und dass er somit auseinandergestreckt bleibt, wenn an den beiden Enden gezogen wird.

2. Operationsinstrument, das zum Implantieren der suburethralen Schlinge von Anspruch 1 bei der Behandlung einer Belastungsharninkontinenz aufgrund einer hypermobilen Harnröhre angeordnet ist, **dadurch gekennzeichnet, dass** es eine Klemme (12) mit einer Länge von ungefähr 15 bis 20 cm zum Positionieren des Bands oder Netzes (1) umfasst, wobei die Klemme (12) unter einem Winkel zwischen 30° und 45° an ihrem distalen Ende (13) gekrümmt ist, mit einer konischen Spitze (15) und einer Öffnung (14) mit einem Durchmesser zwischen 3 und 5 mm und in einem Abstand von 10 mm zum Ende (16).

3. Operationsinstrument, das zum Implantieren der suburethralen Schlinge von Anspruch 1 bei der Behandlung weiblicher Harninkontinenz aufgrund eines angeborenen Mangels des Schließmuskels oder aufgrund einer hypermobilen Harnröhre angeordnet ist, **dadurch gekennzeichnet, dass** es eine kleinkalibrige und gekrümmte wiederverwendbare Metall- oder Einweg-Kunststoff-Nadel (17) aufweist, bei der die distalen 3 bis 4 mm in einer entweder geraden (21) oder leicht gewölbten (22) Form gebogen sind, mit einer konischen Spitze (23) und mit einer Öse (19), deren proximales Ende eine Ausbuchtung oder einen Griff (20, 26, 27) zum Halten der Nadel aufweist.

4. Operationsinstrument nach Anspruch 3, wobei die gebogene Nadel in der Nähe des Griffs eine Biegung von 40° bis 60° bezogen auf ihre Achse aufweist; und wobei sie Mittel (20, 26, 27) zum Fassen oder Fixieren des Fadens (11) aufweist, mit dem die Enden des Bands oder Netzes (1) befestigt werden.

5. Operationsinstrument nach Anspruch 4, wobei die Mittel (20, 26, 27) zum Fassen oder Fixieren des Fadens (11) die Form einer Klemme (28) oder Klammer (29) aufweisen.

6. Operationsinstrument nach Anspruch 4, wobei die Mittel zum Fassen oder Fixieren (20, 26, 27) des Fadens (11) durch einen Schlitz (30) in einem proximalen oder distalen Teil des Steuervorrichtungsgriffs (20) oder einen gebogenen Stift (31) definiert sind, der mit einem Loch (32) versehen sein kann.

## Revendications

1. Ruban sous-urétral pour le traitement chirurgical de l'incontinence urinaire chez la femme, conçu pour fixer l'urètre moyen, le col vésical et l'urètre proximal, afin d'empêcher des fuites d'urine pendant les contraintes et le ruban comprenant une bande ou une maille monofilament ou multifilament (1) de polypropylène ou d'un autre matériau, macroporeux ou similaire, dont la longueur correspond au fascia endo-pelvien auquel il est fixé, sa zone centrale présente deux bords (2) lisses et un filament de soie noire (10) qui traverse la bande ou la maille (1) au centre afin de marquer son point médian ; et dans lequel, le reste de la bande ou de la maille (1) étant conçu comme une pointe de flèche ; et dans lequel la bande ou la maille (1) borde la pointe dans une direction oblique vers le centre, sous la forme de marches ou de dents soit séparées (4, 6) ou contiguës (8, 9), d'angles différents, selon le cas ;
**caractérisé en ce que**
la bande ou la maille (1) comprend un fil monofilament (11) entrelacé de 7 à 15 mm depuis les deux extrémités de la bande ou de la maille (1), pour être tiré et régler la tension de la maille (1), corrigeant l'incontinence urinaire d'effort ;
et dans lequel le filament de soie noire (10) reste fixé aux bords (2) afin de permettre l'extension transversale de la bande ou de la maille (1) ;
et dans lequel le filament de soie noire (10) est conçu pour l'empêcher de se plier sur lui-même avec ses bords longitudinaux s'approchant l'un de l'autre et restant ainsi écartés lorsque les deux extrémités sont tirées.

2. Instrument chirurgical conçu pour implanter le ruban sous-urétral selon la revendication 1 dans le traitement de l'incontinence urinaire d'effort en raison d'une hypermobilité urétrale **caractérisé en ce qu**'il comprend une pince (12) d'environ 15 à 20 cm de longueur pour positionner la bande ou la maille (1), la pince (12) étant incurvée avec un angle compris entre 30° et 45° au niveau de son extrémité distale (13), avec une pointe conique (15) et un orifice (14) avec un diamètre compris entre 3 à 5 mm et 10 mm depuis son extrémité (16).

3. Instrument chirurgical conçu pour implanter le ruban sous-urétral selon la revendication 1 dans le traitement de l'incontinence urinaire chez la femme en raison d'un déficit du sphincter intrinsèque ou en raison d'une hypermobilité urétrale **caractérisé en ce qu'**il comprend une aiguille métallique réutilisable ou en plastique jetable (17), de petit calibre et incurvée, avec son extrémité distale courbée de 3 à 4 mm dans une forme qui est soit droite (21) ou légèrement arquée (22), avec une pointe conique (23) et avec un orifice (19), ayant son extrémité proximale avec un renflement ou une poignée (20, 26, 27) pour maintenir l'aiguille.

4. Instrument chirurgical selon la revendication 3, dans lequel l'aiguille arquée a à proximité de la poignée une courbure de 40° à 60° par rapport à son axe ; et dans lequel elle comprend des moyens (20, 26, 27) pour piéger ou fixer le fil (11) qui fixent les extrémités de la bande ou de la maille (1).

5. Instrument chirurgical selon la revendication 4, dans lequel les moyens (20, 26, 27) pour piéger ou fixer le fil (11) ont la forme d'une attache (28) ou d'une cheville (29).

6. Instrument chirurgical selon la revendication 4, dans lequel les moyens pour piéger ou fixer (20, 26, 27) le fil (11) sont définis par une fente (30) dans une partie proximale ou distale de la poignée du dispositif de commande (20), ou un pivot arqué (31) qui peut être pourvu d'un trou (32).
